# EUROPEAN PATENT SPECIFICATION

(11) **EP 0 743 084 B1**
(45) Date of publication and mention of the grant of the patent: **22.03.2000**
(21) Application number: 96107794.8
(22) Date of filing: 15.05.1996
(51) Int. Cl.: B01D 15/00, B01J 20/24, B01J 20/32, B01D 15/08, A61M 1/36, B01J 20/22

(54) **Dialysis filter/reactor for capturing endotoxins, and relative fabrication method**
Dialysefilter/-reaktor zur Entfernung von Endotoxinen und Verfahren zur Herstellung des Filters/Reaktors
Filtre/réacteur de dialyse pour capter des endotoxines et une méthode de fabrication

(30) Priority: 16.05.1995 IT TO950387
(43) Date of publication of application: 20.11.1996
(73) Proprietor: BELLCO S.p.A., 41037 Mirandola (IT)
(72) Inventor: Tetta, Ciro, 41037 Mirandola (IT); Gervasio, Renzo, 10132 Torino (IT); Wratten, Mary Lou, 41036 Medolla (IT)
(74) Representative: Plebani, Rinaldo

(56) References cited:
- EP-A- 0 034 304
- EP-A- 0 312 104
- EP-A- 0 328 256
- EP-A- 0 424 672
- DE-A- 4 113 602
- US-A- 3 947 352
- US-A- 4 637 880
- US-A- 4 661 260

## Description

The present invention relates to a dialysis filter for extracorporeal treatment of human whole blood or plasma, and which at the same time acts as a reactor for capturing any endotoxins in the blood or plasma. More specifically, the present invention relates to a dialysis filter/reactor wherein endotoxins are captured by an active peptide (polymyxin) immobilized on the walls of the filter contacting the blood or plasma, and which is used for renal support and/or treatment of patients suffering from septic shock.

The present invention also relates to a method of fabricating such a filter/reactor, and more specifically to a method of immobilizing polymyxin inside hollow cellulose fibers with no alteration in the physical-mechanical characteristics of the fibers.

Despite extensive use of antibiotics, septic syndromes still remain a major cause of disease and death, and, during such syndromes, it is vital not only to fight the cause of the infection, but also to provide the patient with general systemic support, particularly renal support. Septic syndromes, also known as "septic shock", are characterized by a drastic reduction in systemic vascular resistance, myocardial depression, and diffused organ failure, the main cause of which lies in infectious agents (generally bacteria) releasing the endotoxins on their cell walls into the patient's bloodstream. One possible supportive therapy is to inject the patient with an antibiotic comprising a peptide - polymyxin (PMX-B) - capable of bonding the endotoxins. This, however, can only be done in small doses and with extreme care, on account of the highly toxic nature of polymyxin at renal level.

One solution proposed and tested to overcome this drawback [W.G.Cheadle et al. - SURGERY 1991; 110:785-92] is to treat the blood of septic shock patients extracorporeally by hemoperfusion through columns/filters in which polymyxin is covalently bonded to insoluble polystyrene fibers (PMX-F) according to a Japanese process developed by TORAY. Despite commercial application, wherein hemoperfusion is performed through a cartridge comprising a number of polystyrene pellets on which the polymyxin is immobilized, this system presents several drawbacks, foremost of which is the fact that it only applies in the main to plasma.

As stated, in fact, in the above and other publications [W.G.Cheadle et al. - SURGERY 1991; 110:785-92], when applied to whole blood, extensive thrombosis phenomena of the PMX-F matrix occur unless accompanied by parallel supportive therapy using heparin or other anticoagulants, which, however, are particularly dangerous and may result in extensive hemorrhaging in the case of septic shock patients.

German Patent DE-A-4113602, on the other hand, relates to the removal of endotoxins by extracorporeal hemo- or plasmaperfusion using columns or capsules, wherein a matrix defined by flat porous cellulose-based sheets immobilizes an endotoxin-capturing agent comprising polyethylimine, which is immobilized on the cellulose support either by adsorption or indirectly by ionic bonding using supports comprising anionic cellulose derivatives. Polymyxin-based systems, however, would appear more effective.

Other similar systems for removing endotoxins or other harmful substances by using a capturing agent immobilized on a fiber support (of different kinds), are disclosed, for example, in EP-A-0328256, EP-A-0312104 and US-A-4637880: all these systems, however, are not indicated for treating patients subject to a septic shock, since they are not completely effective in removing undesirable components in very short treating times and, above all, they do not have ultrafiltering capacities, so as they must be coupled to ultrafiltering units.

Italian Patent Applications n. 67212-A/80 and 67023-A/81 (EP-A-0 034 304) relate to methods of immobilizing peptides comprising hepatic enzymes, in particular Glutathione-S-Transferase, inside hollow cellulose fibers of the type used in hemodialysis filters. This is done by forming covalent bonds between the amino groups of the enzyme and the aldehyde groups of the cellulose - such as Schiff bases - by activating the aldehyde groups with an oxidant, and subsequently stabilizing the bond with a reducer.

Both these patents highlight the importance of selecting the right process conditions for satisfactory results to be obtained (immobilization of a considerable amount of enzyme, long-term immobilization stability, and no change in the cellulose structure, permeability or mechanical strength of the fibers). In particular, the action of the oxidant (sodium periodate) must be carefully timed, in that, over and above 70-80 minutes, the fibers are so oxidized as to be unusable as filters.

It is an object of the present invention to provide a system for the systemic support, in particular renal support, and/or treatment of patients affected with septic shock, and which is based on simultaneously removing endotoxins and any dialyzable harmful substances extracorporeally and directly from whole blood. It is a further object of the present invention to enable the use, with only minor changes, of conventional currently marketed dialysis equipment, and to provide a highly reliable, relatively low-cost system by which absolutely no potentially harmful substances are released into the patient's bloodstream.

According to the present invention, there is provided a dialysis filter/reactor as claimed in claim 1.

Such a dialysis filter/reactor provides simultaneously for extracorporeal ultrafiltration and dialysis of circulating whole blood or plasma, and for capturing and removing any endotoxins in said blood or plasma. According to a further aspect of the present invention, such a filter is fabricated using a method as claimed in claim 4.

In practice, polymyxin is highly satisfactorily immobilized inside hollow cellulose dialysis fibers, and with no impairment in the physical-mechanical characteristics of the fibers, by applying, albeit with a few substantial variations as regards process parameter values, substantially the same method traditionally used to immobilize hepatic enzymes on hollow fibers of the same type.

It is important to stress that, nowhere in existing literature, was a specialist in this particular field given to understand the possibility of safely and effectively immobilizing polymyxin on hollow cellulose hemodialysis fibers without affecting the other properties of the fibers. Indeed, known polymyxin immobilizing techniques deal with entirely different types of supports (polystyrene) and are clearly inapplicable to cellulose materials. When using a cellulose support, existing literature has always clearly discouraged the use of polymyxin, and advocated the use of other substances as endotoxin bonding agents.

Finally, known methods of immobilizing hepatic enzymes on cellulose fibers are first of all applied to a particular class of peptides with physical-chemical characteristics differing widely from those of polymyxin. Moreover, experiments conducted of known methods of immobilizing enzymes on cellulose have shown the impossibility of determining beforehand the feasibility of the process using another peptide. That is, whereas the possibility of achieving a given immobilization is indeed predictable, there is absolutely no guarantee of also maintaining the physical-mechanical characteristics of the support, owing to the impossibility of predicting the operating parameters required to effectively immobilize the desired substance.

In the case of the present invention, for example, extensive testing showed that, to satisfactorily immobilize polymyxin, activation could not be achieved using existing hepatic enzyme techniques, and that recourse would have to be made to prolonged fiber-oxidant contact times, even falling within a range explicitly ruled out in existing literature as being capable of irreparably damaging the structure of cellulose fibers. The method according to the present invention, in fact, employs contact times well in excess of the 70 minutes considered maximum by the known state of the art, and, again with no assistance from existing literature, compensates prolonged contact time with very high (i.e. "fast") specific flow rates - equal to over three times the maximum rates in existing literature - and relatively low oxidant concentrations (though still within the accepted limits).

A dialysis filter/reactor in accordance with the present invention is therefore characterized by comprising a bundle of hollow fibers made of cellulose-based ultrafiltering material, preferably CUPROPHAN™, and on the inner lateral surface of which polymyxin is immobilized stably and unreleasably in use; and at the same time presents substantially the same hydraulic permeability as a normal dialysis filter of cellulose fiber with the same physical-mechanical characteristics.

Tests have also shown that a dialysis filter/reactor in accordance with the present invention provides for removing over 90% of the endotoxins present in a stream of solution or plasma or whole blood contaminated with 50 EU/ml of endotoxins.

The immobilizing step is normally preceded by a conditioning step wherein the fibers are conditioned by feeding through them an 8.4 pH bicarbonate solution. Similarly, the activating step is preceded by a wash step using sterile distilled water, in the course of which, the cellulose fibers absorb water to enable the activating solution to penetrate inside the micropores of the fibers by straightforward diffusion. The final stabilizing step is as usual, and comprises circulating a sterile aqueous solution of sodium borohydride with a concentration of 1 gr/l through the filter fibers for at least 2 hours.

Before being fed through the fibers, all the solutions used in the method according to the present invention are fed through a depyrogenating filter under a sterile hood. Moreover, by virtue of merely feeding and partly or fully recirculating various solutions inside the fibers, the entire method according to the invention may be applied to a normal dialysis filter ready for use, and possibly already installed in a known dialysis machine or specially constructed hydraulic circuit.

A number of non-limiting embodiments of the present invention will be described by way of example with reference to the accompanying drawings, in which:
Figures 1 and 2 show the calibration curves used to test endotoxin removal from an aqueous solution and plasma;
Figure 3 shows the hydraulic permeability characteristics of dialysis filters with and without the treatment according to the present invention.

### EXAMPLE 1

### - Polymyxin immobilization

Three identical filters, each comprising 100 hydraulically parallel hollow CUPROPHAN™ fibers with an inside diameter of 200 µm (transverse through surface of each filter 3.14 mm²), are fitted in parallel to a hydraulic test circuit including a reservoir, a pump, and an HFTO2 depyrogenating filter; and each filter is fitted in series to a single-pass line connected to the reservoir and presenting a recirculating bypass controlled by a Klemmer valve.
The filters are then treated as described below, the reservoir being filled with the various solutions, which are circulated through the filters at a rate of 5 ml/minute unless otherwise specified, and the solution, when recirculated, being fed back to the reservoir for further passage through the filters:
1. With the recirculating bypasses closed (single-pass), the filters are washed in parallel with 400 ml of filtered, sterile distilled water.
2. The circuit is emptied and the line filled with 150 ml of a 2.5 gr/l solution of NaIO₄, which is recirculated inside the filters for 1.5 hours.
3. The filters are washed with 800 ml of filtered sterile water.
4. The oxidant residue is neutralized with 300 ml of a 20% by volume aqueous solution of glycerol, 90% of which is supplied in a single pass, and 10% of which is recirculated.
5. The filters are conditioned with 60 ml of an 8.4 pH aqueous solution of NaHCO₃, 10% of which is recirculated for 5 minutes.
6. The circuit is emptied; the first filter is filled with bicarbonate; and the valves are closed to isolate the relative circuit branch, so that the first filter constitutes the "blank" to which subsequent testing is referred.
7. The lines of the other two filters are filled with 60 ml of an aqueous solution of 3 gr/l of polymyxin in a 0.1 M bicarbonate solution; and the solution, maintained at a temperature of 0 to 4°C, is recirculated through the filters for 12 hours.
8. The recirculated solution is collected; a 1 gr/l concentration of sodium borohydride is added to the recirculated solution in a sterile vessel; and the resulting solution is recirculated through the filters for 2 hours.
9. The filters are washed with 800 ml of a 7.4 pH buffer solution, of which 80% is supplied in a single pass, 10% is recirculated for 30 minutes, and the remaining 10% is again supplied in a single pass.

### EXAMPLE 2

### - Endotoxin absorption

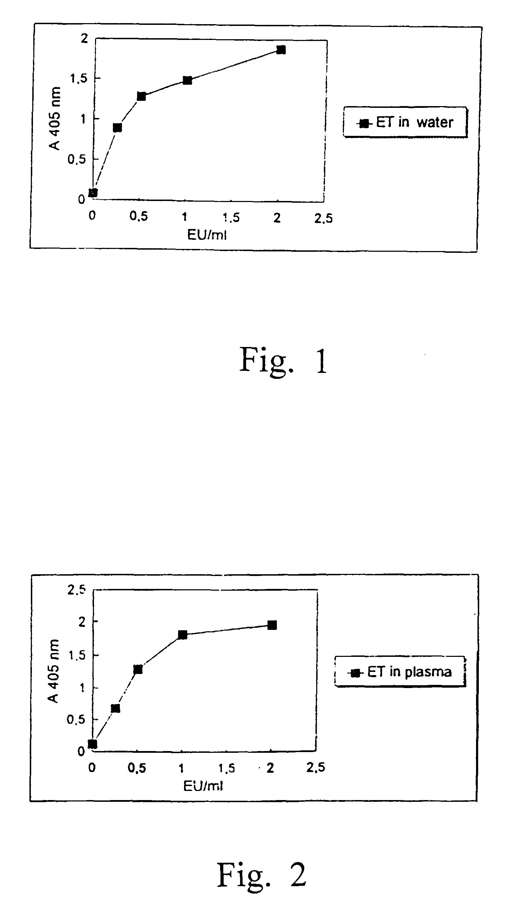

35 ml of a solution of 50 EU/ml of endotoxin in sterile water and apyrogen are prepared, and 10 ml of blood is incubated with 2 µg/ml of endotoxin (25000 EU/ml); an aliquot of the solution at the initial concentration is stored in a refrigerator; an aliquot of blood not incubated with endotoxin (basal) and an aliquot of blood containing 25000 EU/ml of endotoxin are centrifuged, and the surnatant stored in a refrigerator. Using the same circuit as in Example 1, the endotoxin solution is fed through one of the two filters treated with polymyxin (first sample), and the blood incubated with endotoxin is fed through the second (second sample). The filtered endotoxin solution and incubated blood samples are then collected; the incubated blood sample is centrifuged, and the surnatant (plasma) is collected and stored in a refrigerator. The filtered samples of endotoxin solution and blood (centrifuged plasma) at the initial concentrations are then COATEST analyzed graphically against sterile distilled water and apyrogen, and against the plasma derived from the blood not incubated with endotoxin. The calibration curves are shown in Figures 1 and 2.
The test results are shown in Tables 1 and 2.

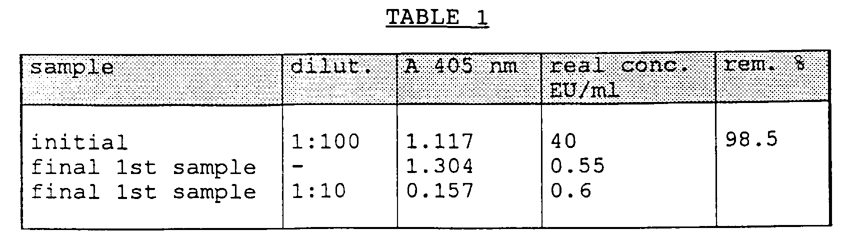

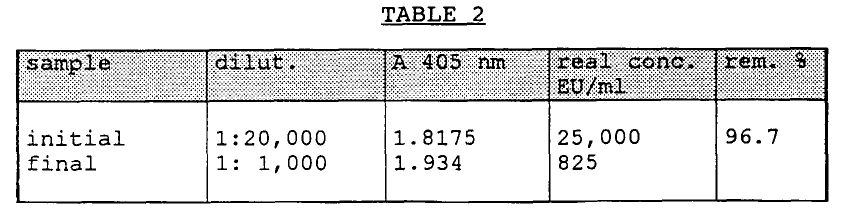

As shown clearly in Tables 1 and 2, over 90% of the endotoxin is removed from both the solution and whole blood. Moreover, in the whole blood test, no coagulation occurred for the first 4 hours. Subsequently, coagulation occurred gradually, with occlusion of the pores, but only very slowly.

### EXAMPLE 3

### - Membrane damage test

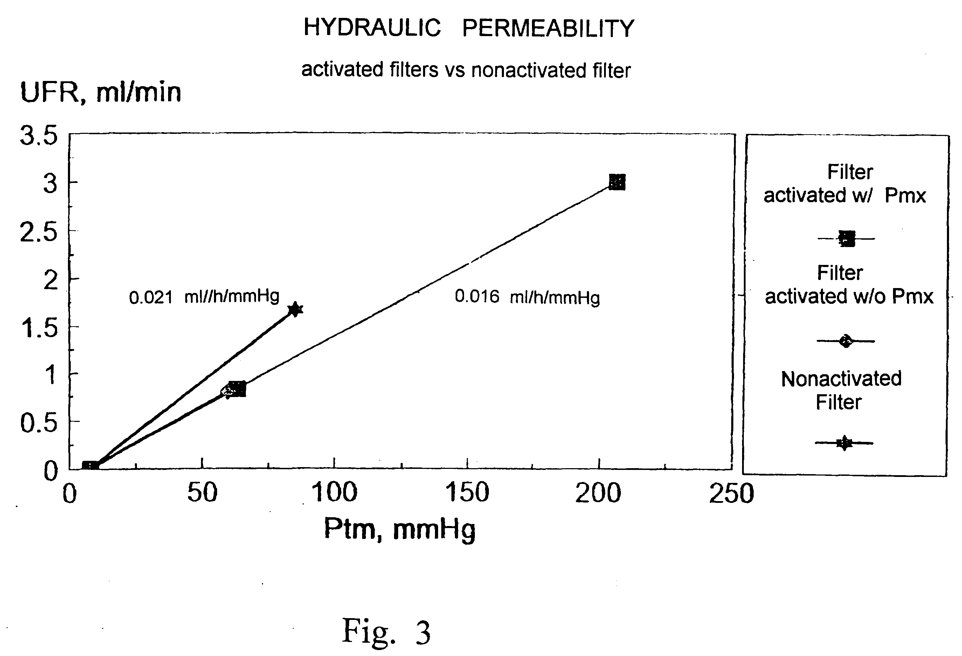

A standard hydraulic permeability test (ultrafiltration versus transmembrane pressure) was conducted of three filters - a blank, sample 1, sample 2 - and of a fourth nontreated, i.e. nonactivated, filter. The results are given in Figure 3, which shows no significant change in permeability.
At the end of the test, each filter was fed with a concentrated BLUE DEXTRAN™ solution (2,000,000 DALTON) and ultrafiltration was enforced. If any of the membranes had been damaged, the colouring agent would have filtered through with the ultrafiltrate. In this particular case, all the ultrafiltrates remained perfectly transparent, indicating absolutely no damage of the membranes treated with polymyxin, even after use. The filters were then washed with deionized water, each was fed with a concentrated COMASSIE BLUE™ solution (861 DALTON), and ultrafiltration was enforced. None of the membranes passed the colouring agent, thus indicating no change in the sieving properties of the membranes treated with polymyxin, even after use (and even using whole blood).
As all the membranes treated with polymyxin were stained blue, the test was repeated using a membrane of HEMOPHAN™, which is known to present amino groups bonded to the cellulose chains. When treated with COMASSIE BLUE™, this membrane also presented a permanent blue colour, which is due to the interaction, known in literature, of the colouring agent molecules with the available protein cation groups. The colouring of the filters treated with polymyxin therefore confirms immobilization of the polymyxin and the extent to which this remains stable even after use.

## Claims

1. A dialysis filter/reactor comprising at least a hollow fiber made of ultrafiltering, cellulose-based material, and an active substance immobilized on the inner lateral surface of said hollow fiber and covalently bonded to the cellulose-based material of the fiber by Schiff bases, wherein said hollow fiber carrying the active substance still presents an ultrafiltering capacity sufficient to act as a dialysis filter; **characterized in that**
(i)- said active substance is a sufficient amount of polymyxin, which has been fixed chemically to said inner lateral surface of said hollow fiber; so that
(ii)- said filter/reactor is capable to provide effective systemic support, in particular renal support, in a patient affected with septic shock by treating an aqueous solution, whole blood and/or plasma coming from said patient by ultrafiltration and, simultaneously, by removing from said aqueous solution, whole blood and/or plasma coming from the patient any endotoxin present therein.

2. A dialysis filter/reactor as claimed in Claim 1, characterized in that the quantity of polymyxin immobilized inside the fibers is such that said filter/reactor is capable of removing over 90% of the endotoxins present in a stream of solution or plasma or whole blood contaminated with 50 EU/ml of endotoxins.

3. A system for systemic support, in particular renal support, and/or treatment of patients affected with septic shock, **characterized by comprising** a dialysis equipment in which the dialysis filter has been replaced by a filter/reactor as claimed in claim 1 or 2.

4. A method of fabricating a dialysis filter/reactor for extracorporeal ultrafiltration and dialysis of circulating whole blood or plasma, and furthermore able to simultaneously capture and remove any endotoxins present in said blood or plasma; said method comprising:
- an activating step wherein a dialysis filter of hollow cellulose-based fibers is activated by circulating a relatively low concentration solution of an oxidant comprising sodium periodate inside said hollow fibers;
- an immobilizing step, subsequent to said activating step, wherein a solution of an active substance to be immobilized is circulated inside said hollow fibers; and
- a stabilizing step, subsequent to said immobilizing step, wherein a solution of a reducing agent comprising sodium borohydride is circulated inside said hollow fibers; **characterized in that**
(i)- said active substance is polymyxin which is circulated inside said hollow fibers, during said immobilization step, as an aqueous solution at a relatively high concentration of polymyxin in 0.1 M bicarbonate of at least 3 grams/liter;
(ii)- said activating step is carried out using said solution of sodium periodate at a concentration of about 2.5 gr./l, and by feeding said solution inside the hollow cellulose fibers at a specific flow rate of at least 1,500 mm³/minute/mm² of fiber section for a period of time of over 70 minutes.

5. A method as claimed in Claim 4, characterized in that said activating step comprises recirculating said oxidizing solution of sodium periodate through said hollow fibers for 1.5 hours at ambient temperature.

6. A method as claimed in anyone of the foregoing Claims 4 and 5, characterized in that said activating step is followed by a neutralizing step wherein any oxidant residue is neutralized by circulating a sterile aqueous solution of 20% by volume of glycerol through said fibers.

7. A method as claimed in one of the foregoing Claims from 4 to 6, characterized in that said immobilizing and stabilizing steps are performed by feeding the respective said solutions through the hollow fibers at the same specific flow rate as for the activating step.

8. A method as claimed in one of the foregoing Claims from 4 to 7, characterized in that said immobilizing step is performed by feeding through the fibers said aqueous solution of polymyxin in 0.1 M bicarbonate, at a temperature ranging between 0 and 4°C, and by recirculating said solution for at least 12 hours.

9. A method as claimed in Claim 8, characterized in that the immobilizing step is preceded by a conditioning step wherein the fibers are conditioned by feeding through them an 8.4 pH solution of bicarbonate.

10. A method as claimed in one of the foregoing Claims from 4 to 9, characterized in that said stabilizing step comprises circulating through said fibers a sterile aqueous solution of sodium borohydride with a concentration of 1 gr./l for at least 2 hours.

11. A method as claimed in one of the foregoing Claims from 4 to 10, characterized in that, before being fed through said fibers, all said solutions are fed through a depyrogenating filter.

## Patentansprüche

1. Dialysefilter/-reaktor, umfassend mindestens eine hohle Faser, hergestellt aus einem auf Cellulose basierenden Ultrafiltrationsmaterial, und eine aktive Substanz, die auf der inneren lateralen Oberfläche der hohlen Faser immobilisiert ist und durch Schiff'sche Basen an das auf Cellulose basierende Material der Faser gebunden ist, wobei die hohle Faser, welche die aktive Substanz trägt, dennoch ein Ultrafiltrationsvermögen aufweist, das ausreichend ist, um als ein Dialysefilter zu wirken; **dadurch gekennzeichnet, daß**
(i) die aktive Substanz eine ausreichende Menge an Polymyxin ist, welches chemisch an der inneren lateralen Oberfläche der hohlen Faser fixiert wurde; so daß
(ii) der Filter/Reaktor in der Lage ist, eine wirksame systemische Unterstützung, insbesondere eine Unterstützung der Niere, bei einem Patienten mit einem septischen Schock zur Verfügung zu stellen durch Behandeln einer wäßrigen Lösung, Vollblut und/oder Plasma, die von dem Patienten stammen, mittels Ultrafiltration und gleichzeitig durch Entfernen von jeglichem darin vorkommenden Endotoxin aus der wäßrigen Lösung, dem Vollblut und/oder Plasma, die von dem Patienten stammen.

2. Dialysefilter/-reaktor nach Anspruch 1, dadurch gekennzeichnet, daß die Menge an im Inneren der Fasern immobilisiertem Polymyxin derart ist, daß der Filter/Reaktor in der Lage ist, über 90% der Endotoxine zu entfernen, die in einem Strom einer Lösung oder eines Plasmas oder von Vollblut, kontaminiert mit 50 EU/ml an Endotoxinen, vorhanden sind.

3. System zur systemischen Unterstützung, insbesondere einer Unterstützung der Niere, und/oder Behandlung von Patienten mit einem septischen Schock, **dadurch gekennzeichnet, daß** es eine Dialyseausrüstung umfaßt, in welcher der Dialysefilter durch einen Filter/Reaktor gemäß Anspruch 1 oder 2 ersetzt wurde.

4. Verfahren zur Herstellung eines Dialysefilters/-reaktors für eine extrakorporale Ultrafiltration und Dialyse von zirkulierendem Vollblut oder Plasma, welches darüber hinaus in der Lage ist, gleichzeitig jegliche in dem Blut oder Plasma vorhandene Endotoxine einzufangen und zu entfernen; wobei das Verfahren folgendes umfaßt:
- einen Aktivierungsschritt, in welchem ein Dialysefilter aus hohlen auf Cellulose basierenden Fasern aktiviert wird, indem eine Lösung mit einer relativ geringer Konzentration eines Oxidationsmittels, welches Natriumperiodat umfaßt, innerhalb der hohlen Fasern zirkuliert wird;
- einen Immobilisierungsschritt, der dem Aktivierungsschritt nachfolgt, in welchem eine Lösung einer zu immobilisierenden aktiven Substanz innerhalb der hohlen Fasern zirkuliert wird; und
- einen Stabilisierungsschritt, der dem Immobilisierungsschritt nachfolgt, in welchem eine Lösung eines Reduktionsmittels, das Natriumborhydrid umfaßt, innerhalb der hohlen Fasern zirkuliert wird;
**dadurch gekennzeichnet, daß**
(i) die aktive Substanz Polymyxin ist, welches innerhalb der hohlen Fasern während des Immobilisierungsschrittes als eine wäßrige Lösung mit einer relativ hohen Konzentration an Polymyxin von mindestens 3 g/l in 0,1 M Bicarbonat zirkuliert wird;
(ii) der Aktivierungsschritt durchgeführt wird unter Verwendung der Lösung von Natriumperiodat bei einer Konzentration von ungefähr 2,5 g/l und durch Zuführen dieser Lösung in die hohlen Cellulosefasern mit einer spezifischen Durchflußrate von mindestens 1500 mm³/Minute/mm² eines Faserabschnitts während eines Zeitraums von über 70 Minuten.

5. Verfahren nach Anspruch 4, dadurch gekennzeichnet, daß der Aktivierungsschritt ein Rezirkulieren der oxidierenden Lösung von Natriumperiodat während 1,5 Stunden bei Umgebungstemperatur durch die hohlen Fasern umfaßt.

6. Verfahren nach einem der vorhergehenden Ansprüche 4 und 5, dadurch gekennzeichnet, daß dem Aktivierungsschritt ein Neutralisierungsschritt nachfolgt, in welchem jeglicher Oxidationsmittelrückstand neutralisiert wird durch Zirkulieren einer sterilen wäßrigen Lösung von Glycerol mit 20 Vol.-% durch die Fasern.

7. Verfahren nach einem der vorhergehenden Ansprüche 4 bis 6, dadurch gekennzeichnet, daß die Immobilisierungs- und Stabilisierungsschritte durchgeführt werden durch Zuführen der entsprechenden Lösungen durch die hohlen Fasern bei derselben spezifischen Durchflußrate wie bei dem Aktivierungsschritt.

8. Verfahren nach einem der vorhergehenden Ansprüche 4 bis 7, dadurch gekennzeichnet, daß der Immobilisierungsschritt durchgeführt wird durch Hindurchleiten der wäßrigen Lösung an Polymyxin in 0,1 M Bicarbonat durch die Fasern bei einer Temperatur, die zwischen 0 und 4°C liegt, und durch Rezirkulieren der Lösung während mindestens 12 Stunden.

9. Verfahren nach Anspruch 8, dadurch gekennzeichnet, daß dem Immobilisierungsschritt ein Konditionierungsschritt vorausgeht, in welchem die Fasern konditioniert werden mittels Durchleiten einer Lösung von Bicarbonat mit einem pH von 8,4.

10. Verfahren nach einem der vorhergehenden Ansprüche 4 bis 9, dadurch gekennzeichnet, daß der Stabilisierungsschritt ein Zirkulieren einer sterilen wäßrigen Lösung von Natriumborhydrid mit einer Konzentration von 1 g/l während mindestens 2 Stunden durch die Fasern umfaßt.

11. Verfahren nach einem der vorhergehenden Ansprüche 4 bis 10, dadurch gekennzeichnet, daß vor dem Durchleiten durch die Fasern alle Lösungen durch einen Depyrogenierungsfilter geleitet werden.

## Revendications

1. Filtre/réacteur de dialyse comprenant au moins une fibre creuse constituée d'une matière d'ultrafiltration à base de cellulose, et d'une substance active immobilisée sur la surface latérale intérieure de ladite fibre creuse et liée par covalence à la matière à base de cellulose de la fibre par des bases de Schiff, dans lequel ladite fibre creuse portant la substance active présente encore une capacité d'ultrafiltration suffisante pour agir comme filtre de dialyse ; caractérisé en ce que
(i) ladite substance active est une quantité suffisante de polymyxine, qui a été fixée chimiquement à ladite surface latérale intérieure de ladite fibre creuse ; en sorte que
(ii) ledit filtre/réacteur est capable d'offrir une assistance systémique efficace, en particulier une assistance rénale, à un patient atteint d'un choc septique en traitant par ultrafiltration une solution aqueuse, du sang entier et/ou du plasma provenant dudit patient et, simultanément, en retirant de ladite solution aqueuse, dudit sang entier et/ou dudit plasma provenant du patient toute endotoxine qui y est présente.

2. Filtre/réacteur de dialyse tel que revendiqué dans la revendication 1, caractérisé en ce que la quantité de polymyxine immobilisée à l'intérieur des fibres est telle que ledit filtre/réacteur est capable d'éliminer plus de 90 % des endotoxines présentes dans un courant de solution ou de plasma ou de sang entier contaminé avec 50 UE/ml d'endotoxines.

3. Système d'assistance systémique, en particulier d'assistance rénale, et/ou de traitement de patients atteints de choc septique, caractérisé en ce qu'il comprend un équipement de dialyse dans lequel le filtre de dialyse a été remplacé par un filtre/réacteur tel que revendiqué dans la revendication 1 ou 2.

4. Procédé de fabrication d'un filtre/réacteur de dialyse pour l'ultrafiltration et la dialyse extra-corporelles de sang entier ou de plasma circulant, et capable en outre de capturer et d'éliminer simultanément toutes endotoxines présentes dans ledit sang ou plasma, ledit procédé comprenant :
- une étape d'activation dans laquelle un filtre de dialyse constitué de fibres creuses à base de cellulose est activé par circulation à l'intérieur desdites fibres creuses d'une solution à relativement basse concentration d'un oxydant comprenant du periodate de sodium ;
- une étape d'immobilisation, postérieure à ladite étape d'activation, dans laquelle une solution d'une substance active à immobiliser est mise en circulation à l'intérieur desdites fibres creuses ; et
- une étape de stabilisation, postérieure à ladite étape d'immobilisation, dans laquelle une solution d'un agent réducteur comprenant du borohydrure de sodium est mise en circulation à l'intérieur desdites fibres creuses ; caractérisé en ce que
(i) ladite substance active est une polymyxine qui est mise en circulation à l'intérieur desdites fibres creuses, pendant ladite étape d'immobilisation, sous forme d'une solution aqueuse à une concentration relativement élevée de polymyxine d'au moins 3 grammes/litre dans du bicarbonate 0,1M ;
(ii) ladite étape d'activation est effectuée en utilisant ladite solution de periodate de sodium à une concentration d'environ 2,5 g/litre et en faisant passer ladite solution à l'intérieur des fibres creuses de cellulose à un débit spécifique d'au moins 1500 mm³/min/mm² de section de fibre pendant une période de temps supérieure à 70 minutes.

5. Procédé tel que revendiqué dans la revendication 4, caractérisé en ce que ladite étape d'activation comprend un recyclage de ladite solution oxydante de periodate de sodium à travers lesdites fibres creuses pendant 1,5 heure à la température ambiante.

6. Procédé tel que revendiqué dans l'une quelconque des revendications 4 et 5 précédentes, caractérisé en ce que ladite étape d'activation est suivie d'une étape de neutralisation dans laquelle tout résidu d'oxydant est neutralisé par circulation d'une solution aqueuse stérile à 20 % en volume de glycérol à travers lesdites fibres.

7. Procédé tel que revendiqué dans l'une des revendications 4 à 6 précédentes, caractérisé en ce que lesdites étapes d'immobilisation et de stabilisation sont exécutées en faisant passer lesdites solutions respectives à travers les fibres creuses au même débit spécifique que pour l'étape d'activation.

8. Procédé tel que revendiqué dans l'une des revendications 4 à 7 précédentes, caractérisé en ce que ladite étape d'immobilisation est exécutée en faisant passer à travers les fibres ladite solution aqueuse de polymyxine dans du bicarbonate 0,1M à une température comprise entre 0 et 4°C et en recyclant ladite solution pendant au moins 12 heures.

9. Procédé tel que revendiqué dans la revendication 8, caractérisé en ce que l'étape d'immobilisation est précédée d'une étape de conditionnement dans laquelle les fibres sont conditionnées en faisant passer à travers les fibres une solution de bicarbonate à pH 8,4.

10. Procédé tel que revendiqué dans l'une des revendications 4 à 9 précédentes, caractérisé en ce que ladite étape de stabilisation comprend la circulation à travers lesdites fibres d'une solution aqueuse stérile de borohydrure de sodium d'une concentration de 1 g/litre pendant au moins 2 heures.

11. Procédé tel que revendiqué dans l'une des revendications 4 à 10 précédentes, caractérisé en ce que, avant de les faire passer à travers lesdites fibres, on fait passer toutes lesdites solutions à travers un filtre d'élimination des pyrétogènes.
